Europäisches Patentamt

European Patent Office

Office européen des brevets

(19)

(11) Publication number: **0 269 401**
**A2**

# EUROPEAN PATENT APPLICATION

(12)

(21) Application number: 87310315.4

(22) Date of filing: 23.11.87

(51) Int. Cl.⁴: **A 41 B 13/02**
**A 61 F 13/18**

(30) Priority: 26.11.86 US 935451

(43) Date of publication of application:
01.06.88 Bulletin 88/22

(84) Designated Contracting States:
AT BE CH DE ES FR GB GR IT LI LU NL SE

(71) Applicant: THE PROCTER & GAMBLE COMPANY
One Procter & Gamble Plaza
Cincinnati Ohio 45202 (US)

(72) Inventor: Ahr, Nicholas Albert
3736 Benhill Drive
Cincinnati Ohio 45224 (US)

(74) Representative: Gibson, Tony Nicholas et al
Procter & Gamble (NTC) Limited Whitley Road
Longbenton Newcastle upon Tyne NE12 9TS (GB)

(54) Absorbent garment having air pervious flaps.

(57) An integral disposable absorbent garment, such as a disposable diaper or an incontinent brief, having flaps joined to and extending from and beyond the ends of an absorptive means. The flaps have an air pervious zone which permits air, vapor, and other gases to pass from the interior of the garment and the wearer's skin to the exterior of the garment and the surrounding air. Thus, the article is made cooler in the non-absorptive areas of the article located beyond the ends of the absorptive means, resulting in increased comfort for the wearer.

EP 0 269 401 A2

**Description**

## ABSORBENT GARMENT HAVING AIR PERVIOUS FLAPS

### FIELD OF THE INVENTION

The present invention relates to disposable absorbent garments such as disposable diapers, and more particularly, to absorbent garments having flaps extending from an absorptive means. The flaps are provided with an air pervious zone so as to improve the breathability and as a result increase the wearing comfort of the article.

### BACKGROUND OF THE INVENTION

The major function of absorbent garments such as disposable diapers is to absorb and contain body exudates. Such articles are thus intended to prevent body exudates from soiling, wetting, or otherwise contaminating clothing or other articles, such as bedding, that come in contact with the wearer. It is known that the exterior of disposable diapers can be covered with a flexible and impermeable sheet to prevent any absorbed liquid from passing through the diaper and soiling adjacent articles. These covering sheets are known as backsheets and are generally constructed from a waterproof plastic such as polyethylene. While preventing liquid from passing therethrough and while helping to contain liquid within the diaper, these backsheets, unfortunately, sometimes make the diaper feel hot and uncomfortable to the wearer because they are air, as well as liquid, impermeable. This is most evident in adult size diapers due to the relatively large area of skin covered by the article.

In an effort to provide a cooler and hence a more comfortable garment, breathable backsheets intending to allow the passage of vapor through them while retarding the passage of liquids have been developed. For example, U.S. Patent number 3,881,489, issued to Hartwell on May 6, 1975, teaches a breathable backsheet comprising, in combination, two layers, the first of which is a low void volume perforated thermoplastic film and the second of which is a porous high void volume hydrophobic tissue. U.S. Patent number 3,989, 867, issued to Sisson on November 2, 1976, teaches a breathable backsheet provided with tapered, hollowed bosses which prevent the passage of liquids while allowing vapors to flow readily therethrough. U.S. Patent number 4,341,216, issued to Obenour on July 27, 1982, teaches a two-ply breathable backsheet which extends beyond the ends of an absorbent core, comprising, in combination, an outer sheet being vapor pervious and relatively liquid impervious in a longitudinally extending central region while being totally impervious on either side of the central region, and a totally impermeable inner panel located in the crotch region of the diaper being from about 25% to about 85% of the length of the absorbent core.

While the backsheets discussed above do provide some measure of improvement over the more common impermeable backsheets, and while the last named device is of particular value, the devices fail to adequately address the need for a more efficient transfer of air in those areas of the garment which extend beyond the absorptive part of the garment.

Therefore, it is an object of the present invention to provide an absorbent garment which has improved breathability beyond the ends of the absorptive means. It is an additional object of the present invention to provide an absorbent garment having flaps joined to an absorptive means, the flaps having an air pervious zone.

These and other objects of the present invention will be more readily apparent when considered in reference to the following description and when taken in conjunction with the accompanying drawings.

### SUMMARY OF THE INVENTION

In accordance with the present invention, an integral disposable absorbent garment, such as a diaper, is provided with an absorptive means comprising an absorbent core having a garment surface and a body surface; a liquid impervious backsheet positioned adjacent the garment surface of the absorbent core; and a liquid pervious topsheet positioned adjacent the body surface of the absorbent core. Joined to the absorptive means, and extending beyond the ends thereof, are flaps. The flaps are provided with an air pervious zone. The air pervious zone permits air to pass from the interior of the garment to the exterior of the garment. Thus, the article is made cooler in the non-absorptive areas of the article located beyond the ends of the absorptive means, resulting in increased comfort for the wearer.

### BRIEF DESCRIPTION OF THE DRAWINGS

While the specification concludes with claims particularly pointing out and distinctly claiming the subject matter which is regarded as forming the present invention, it is believed that the invention will be better understood from the following descriptions which are taken in conjunction with the accompanying drawings in which like designations are used to designate substantially identical elements and in which:

Figure 1 is a plan view of a disposable diaper embodiment of the present invention having portions cut away to reveal underlying structure.

Figure 2 is a fragmentary sectional view taken along section line 2-2 of Figure 1.

Figure 3 is a side view of the disposable diaper of Figure 1.

Figure 4, is a side view of another disposable diaper embodiment of the present invention.

Figure 5 is a side view of still another disposable diaper embodiment of the present invention.

Figure 6 is a side view of still another disposable diaper embodiment of the present invention.

Figure 7 is a plan view of yet another disposable diaper embodiment of the present invention.

## DETAILED DESCRIPTION OF THE PREFERRED EMBODIMENTS

The present invention relates to integral disposable absorbent garments such as disposable diapers, and more particularly, to integral disposable absorbent garments comprising an absorptive means and at least one flap joined to and extending beyond a longitudinal end of said absorptive means. The flap has an air pervious zone.

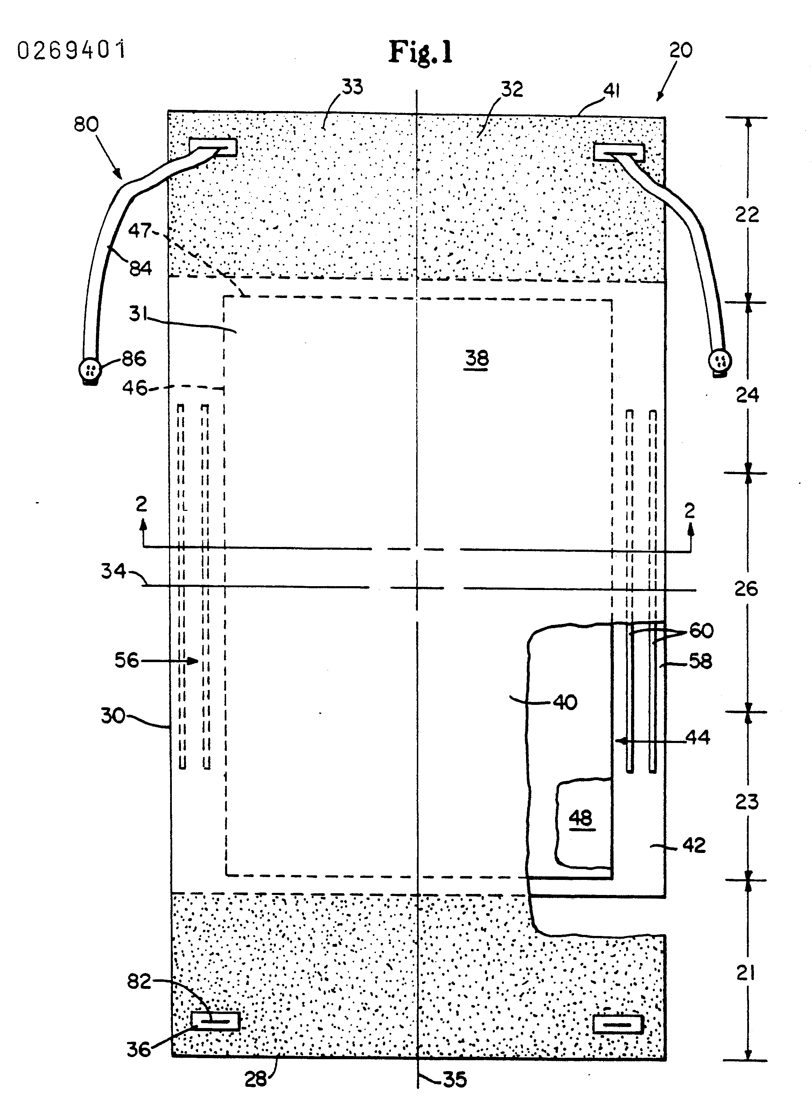

As used herein, the term "integral disposable absorbent garment" refers to articles which absorb and contain body exudates and more specifically refers to garments which are placed against or in proximity to the body of a wearer to absorb and contain the various exudates discharged from the body and which are intended to be discarded after a single use (i.e., they are not intended to be laundered or otherwise restored or reused); and which do not require separate manipulative parts like a separate holder and liner. A preferred embodiment of the integral disposable absorbent garment of the present invention is shown in Figure 1 as it would be used in a diaper 20. As used herein, the term "diaper" refers to a garment generally worn by infants and incontinent persons about the lower torso. It should be understood, however, that the present invention is also applicable to other disposable absorbent garments such as incontinent briefs, and the like.

Referring now to the drawings, and in particular Figure 1 thereof, there is shown a preferred embodiment of the present invention as it would be used in a diaper intended to be worn by an incontinent person. Figure 1 is a partially cut away plan view of the diaper 20 of the present invention in its flat out, uncontracted state (i.e., with all elastic induced contraction pulled out) with the portion of the diaper 20 which contacts the wearer facing the viewer. Basically, the diaper 20 comprises an absorptive means 31 and two flaps 32. The absorptive means may be any means which is generally compressible, conformable, non-irritating to the wearer's skin, and which is capable of absorbing and containing body exudates, such as feces, urine, blood, pus, and the like. In a preferred embodiment, the absorptive means 31 comprises a liquid pervious topsheet 38; a liquid impervious backsheet 42; and an absorbent core 44 positioned between said topsheet 38 and said backsheet 42. The absorbent core 44 has core side edges 46 and core waist edges 47 which define the lateral and longitudinal edges, respectively, of the absorptive means 31. The absorbent core 44 further comprises an absorbent layer 48 and first and second tissue layers 50 and 52, respectively, which envelope the absorbent layer 48.

Joined to the absorptive means 31 and positioned adjacent to the core waist edges 47 of the absorptive core 44 and extending outwardly away therefrom, are the flaps 32. As used herein, the term "joined" includes any means for affixing the flaps 32 to the absorptive means 31, and includes embodiments wherein the flaps 32 are separate members directly or indirectly secured to the absorptive means 31 (i.e., integral) or embodiments wherein the flaps 32 are constructed from the same member or material as an element of the absorptive means 31 so that the flaps 32 are continuous and undivided elements of the absorptive means 31 (i.e., unitary). A flap 32 may be any member or combination of members which is generally conformable and non-irritating to the wearer's skin and has an air pervious zone 33 which allows air, vapor, or other gases to pass from the interior of the garment and the wearer's skin to the exterior of the garment and the surrounding air.

The diaper 20 has a liquid receiving top surface 40 which is generally defined by the topsheet 38 and a back surface 54 which is generally defined by the backsheet 42. Preferably, the topsheet 38 and the backsheet 42 have length and width dimensions generally larger than the absorbent core 44, so that they extend beyond the core side edges 46 and the core waist edges 47 of the absorbent core 44 where they are associated together in a suitable manner. As used herein, the term "associated" encompasses configurations whereby the topsheet 38 is directly joined to the backsheet 42 by affixing the topsheet 38 directly to the backsheet 42 and configurations whereby the topsheet 38 is indirectly joined to the backsheet 42 by affixing the topsheet 38 to intermediate members which in turn are affixed to the backsheet 42. The extension of the topsheet 38 and/or the backsheet 42 beyond the core side edges 46 of the absorbent core 44 forms the longitudinal edges 30 of the diaper 20. The furthest extending ends of the flaps 32 form the end edges 41 of the diaper 20. The end edges 41 and the longitudinal edges 30 of the diaper 20 comprise the periphery 28 of the diaper 20. In a preferred embodiment, the topsheet 38 and the backsheet 42 extend beyond the core side edges 46 of the absorbent core 44 a minimum distance of at least 1.3 cm to 2.5 cm ( 0.5 to 1.0 inch) where they are joined directly to each other in the longitudinal edge 30 of the diaper 20 by attachment means as are known in the art. Also, in a preferred embodiment of the diaper 20, as depicted in Figure 1, the flaps 32 are unitary with the absorptive means 31; the topsheet 38 extending beyond the core waist edges 47 16.25 cm (6.5 inches) while the backsheet 42 terminates 2.5 cm (1.0 inch) beyond the core waist edges 47 where it is associated with the topsheet 38 by attachment means as are known in the art. The attachment means may be, for example, a uniform continuous layer of adhesive, a patterned layer of adhesive, or an array of separate lines or spots of adhesive.

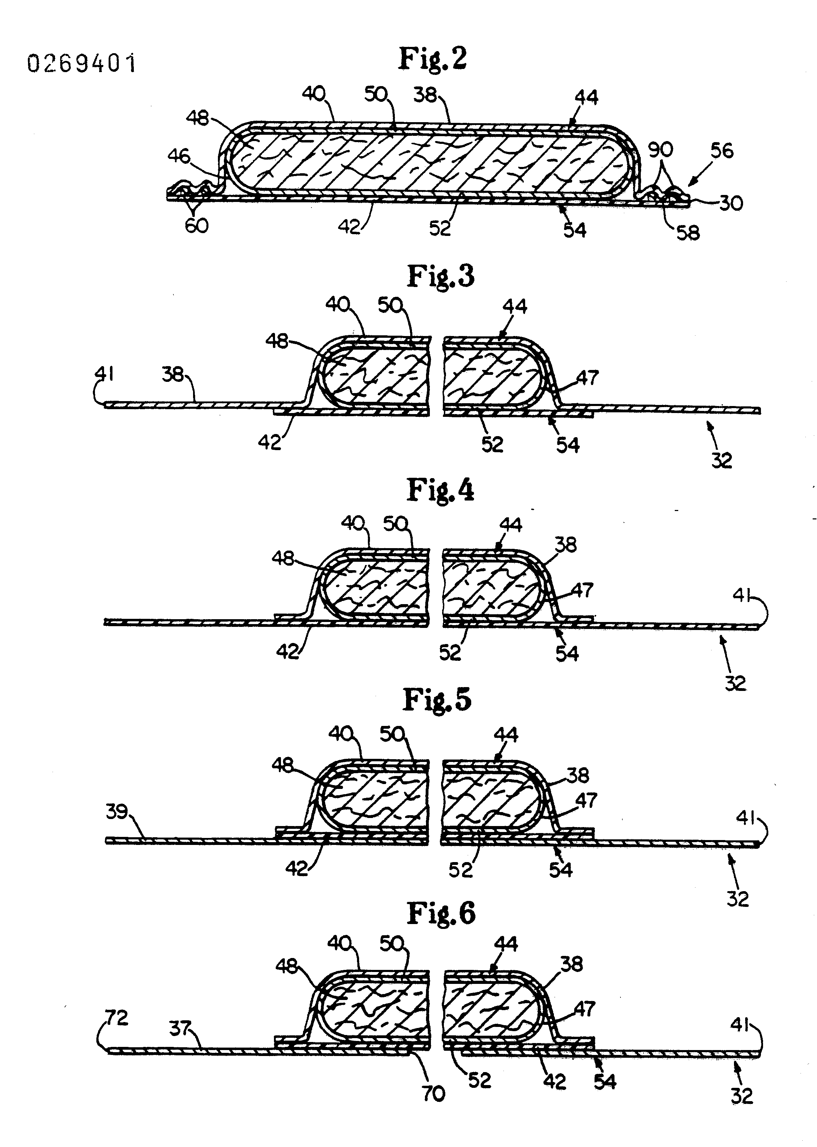

Figure 2 is a fragmentary sectional view taken along line 2-2 of Figure 1 and depicts a preferred diaper construction in the crotch region 26 of the diaper 20. The absorbent core 44 comprises the absorbent layer 48 that is shown as being completely enveloped by the first and second tissue layers 50 and 52. The absorbent core 44 is disposed between the topsheet 38 and the backsheet 42; the backsheet 42, extending beyond the core side edges 46 of the absorbent core 44. The topsheet 38 covers the body surface of the absorbent core 44 and extends beyond the core side edges 46 of the absorbent core 44 inwardly and preferably adjacent the longitudinal edges 30, where it is preferably associated with the backsheet 42.

Looking at some of the elements of the diaper 20 more specifically, the topsheet 38 is positioned adjacent the body surface of the absorbent core 44 and overlays a major portion of the absorbent core 44 so that exudates are discharged onto the topsheet 38 and penetrate through the topsheet 38 where they are absorbed by the absorbent core 44. The topsheet 38 extends outwardly toward the edges of the absorbent core 44 so that a major portion of the absorbent core 44 is disposed between the backsheet 42 and the topsheet 38. In a preferred embodiment as shown in Figure 1, the topsheet 38 has length and width dimensions generally larger than those of the absorbent core 44. The topsheet 38 preferably extends toward the edges of the absorbent core 44, preferably beyond the core waist edges 47 and the core side edges 46 in at least the crotch region 26.

The topsheet 38 is compliant, soft feeling, and non-irritating to the wearer's skin. Further, the topsheet 38 is liquid pervious, permitting liquids to readily penetrate through its thickness. A suitable topsheet may be manufactured from a wide range of materials, such as porous foams, reticulated foams, apertured plastic films, natural fibers (e.g., wood or cotton fibers), synthetic fibers (e.g., polyester or polypropylene fibers) or from a combination of natural and synthetic fibers. Preferably, it is made of hydrophobic material to isolate the wearer's skin from liquids in the absorbent core 44. A particularly preferred topsheet 38 comprises staple length polypropylene fibers of 0.166 Tex such as Hercules Type 151 polypropylene, marketed by Hercules, Inc. of Wilmington, Delaware. As used herein, the term "staple length fibers" refers to those fibers having a length of at least 15.9 mm (0.625 inches).

There are a number of manufacturing techniques which may be used to manufacture the topsheet 38. For example, the topsheet 38 may be woven, non-woven, spunbonded, carded, or the like. A preferred topsheet 38 is carded and thermally bonded by means well known to those skilled in the fabrics art. Preferably, the topsheet 38 has a weight from 18 to 25 grams per square meter, a minimum dry tensile strength of at least 400 grams per centimeter in the machine direction and a wet tensile strength of at least 55 grams per centimeter in the cross machine direction.

The absorbent core 44 may be any means which is generally compressible, conformable, non-irritating to the wearer's skin and capable of absorbing and containing liquids and certain body exudates. A preferred absorbent core 44 has first and second opposed faces (a body surface and a garment surface) and comprises an absorbent layer 48 and first and second tissue layers 50 and 52, respectively. The first and second tissue layers 50 and 52 overlay the major surfaces of the absorbent layer 48 to form the garment surface and the body surface of the absorbent core 44.

The absorbent layer 48 may be manufactured in a wide variety of sizes and shapes (e.g., rectangular, hourglass, etc.) and from a wide variety of liquid absorbent materials commonly used in disposable diapers and other absorbent articles, such as comminuted wood pulp which is generally referred to as airfelt. Examples of other suitable absorbent materials include creped cellulose wadding, absorbent foams, absorbent sponges, super absorbent polymers, absorbent gelling materials, or any equivalent materials or combination of materials. The total absorbent capacity of the absorbent layer 48 should, however, be compatible with the design exudate loading in the intended use of the diaper 20. Further, the size and absorbent capacity of the absorbent layer 48 may be varied to accommodate wearers ranging from infants to adults.

A preferred embodiment of the diaper 20 has a rectangular-shaped absorbent layer 48 and is intended to be worn by adults. The absorbent layer 48 is preferably a batt of airfelt 11.25 cm (4.5 inches) wide (lateral dimension) and 40 cm (16.0 inches) long (longitudinal dimension). The airfelt used in the absorbent layer 48 weighs from 30 grams to 56 grams, has a generally uniform caliper of 1.0 cm (0.40 inch), and has an absorbent capacity of from 8 grams to 16 grams of water per gram of absorbent material. It should be understood, however, that the size, shape, configuration, and total absorbent capacity of the absorbent layer 48 may be varied to accommodate wearers ranging rom infants through adults. Therefore, the dimensions, shape, and configuration of the absorbent layer 48 may be varied (e.g., the absorbent layer may have a varying caliper, or a hydrophilic gradient, or may contain absorbent gelling materials).

The first and second tissue layers 50 and 52 improve the tensile strength of the absorbent core 44 and reduce the tendency of the absorbent layer 48 to split, lump or ball when wetted. The first and second tissue layers 50 and 52 also help to improve lateral wicking of the absorbed exudates, thereby providing a more even distribution of the exudates throughout the absorbent layer 48. While a number of materials and manufacturing techniques may be used to manufacture the first and second tissue layers 50 and 52, satisfactory results have been obtained with sheets of tissue paper having a basis weight of 16 grams per square meter (10 lbs. per 3,000 square feet) and having an air permeability of 30.5 cubic meters per minute per square meter (100 cubic feet per minute per square foot) at a pressure differential of 12.8 millimeters of water (0.5 inch). While the first and second tissue layers 50 and 52 are preferably coterminous with the absorbent layer 48, they may have different dimensions, a different configuration, or they may be omitted entirely.

The backsheet 42 is positioned adjacent the garment surface of the absorbent core 44 and is preferably attached thereto by attachment means (not shown) such as those well known in the art. For example, the backsheet 42 may be secured to the absorbent core 44 by a uniform continuous layer of adhesive, a patterned layer of adhesive, or an array of separate lines or spots of adhesive. Adhesives which have been found to be satisfactory are manufactured by Eastman Chemical Products Company of Kingsport, Tennessee, and marketed under the tradename Eastobond A-3 and by Century Adhesives, Inc., of Columbus, Ohio, and marketed under the tradename Century 5227.

The backsheet 42 is impervious to liquids and is preferably manufactured from a thin plastic film, although other flexible liquid impervious materials may also be used. The backsheet 42 prevents the exudates absorbed and contained in the absorbent core 44 from wetting articles which contact the diaper 20 such as bedsheets and undergarments. Preferably, the backsheet 42 is a polyethylene film having a thickness of from 0.012 mm (0.5 mil) to 0.051 mm (2.0 mils) although other flexible, liquid impervious materials may be used. As used herein, the term "flexible" refers to materials which are compliant and which will readily conform to the general shape and contours of the human body.

A suitable polyethylene film is manufactured by Monsanto Chemical Corporation and marketed in the trade as Film No. 8020. The backsheet 42 is preferably embossed and/or matte finished to provide a more clothlike appearance. Further, the backsheet 42 may permit vapors to escape from the absorbent core 44 while still preventing exudates from passing through the backsheet 42.

The size of the backsheet 42 is dictated by the size of the absorbent core 44 and the exact diaper design selected. In a preferred embodiment, backsheet 42 has a rectangular shape extending beyond the absorbent core 44 a minimum distance of at least 1.3 cm to 2.5 cm (0.5 to 1.0 inch) around the entire core.

The flaps 32 may be any member or combination of members or materials which are generally conformable, non-irritating to the wearer's skin and have an air pervious zone 33. The air pervious zone 33 may comprise discrete and separate areas. Preferably, the flaps 32 are air pervious over their entire area. However, the flaps 32 might be air pervious in only certain zones. For instance, the air pervious zones 33 of the flaps 32 might be located in the center of the flaps 32, or around the perimeter of the flaps 32, or horizontally across the width of the flaps 32, or any other configuration. In a preferred embodiment of the diaper 20, as depicted in Figure 1, the flaps 32 are air pervious over their entire area excluding a 2.5 cm (1.0 inch) by 5 cm (2.0 inches) area in each of the furthest extending corners of each flap 32 where a poly tape reinforcing member 36 is applied, resulting in a total air pervious area of about 308.75 square centimeters (47.5 square inches) for each flap 32. However, in alternatively preferred embodiments the air pervious area is preferably from 6.5 to 455.0 square centimeters (1.0 to 70.0 square inches), and more preferably from 58.5 to at least 455.0 square centimeters (9.0 to 70.0 square inches), and most preferably from 195.0 to 455.0 square centimeters (30.0 to 70.0 square inches). Furthermore, the air pervious zones 33 of the flaps 32 may not be totally air pervious. The air pervious zones 33 of the flaps 32 preferably have an air porosity of at least 400 l/sec/m$^2$, more preferably at least 800 l/sec/m$^2$, and most preferably at least 2,000 l/sec/m$^2$. In a preferred embodiment of the diaper 20 as depicted in Figure 1, the flaps 32 comprise the topsheet 38 and have an air porosity of 6,488 l/sec/m$^2$.

The air porosity of the nonwoven fabrics is determined in accordance with the ASTM test method D-737, with the material to be tested first being conditioned at 23° C.±1° C. and 50%±2% relative humidity for a minimum of 12 hours prior to testing. The air porosity is reported as liters per second per square meter at 12.7 mm H$_2$O differential pressure. A high volume of air passing through the test material is desired.

In a particularly preferred embodiment of the invention, the diaper 20 has a pair of side flaps 58, one or more side flap elastic members 60 associated with each of said side flaps 58, and a pair of gasketing cuffs 56. The side flaps 58 comprise those portions of the diaper 20 between the core side edges 46 and the longitudinal edges 30. The side flap elastic members 60 are operatively associated with the side flaps 58 so that they effectively contract or gather the side flaps 58 to provide gasketing cuffs 56 about the legs of the wearer. While the topsheet 38, the absorbent core 44, the backsheet 42, the side flaps 58, the side flap elastic members 60, and the gasketing cuffs 56 may be assembled in a variety of well known configurations, a preferred diaper configuration is described generally in U.S. Patent 3,860,003 entitled "Contractable Side Portions For Disposable Diaper", which issued to K. B. Buell on January 14, 1975. In addition, a method and apparatus suitable for manufacturing a disposable diaper having elastically contractible gasketing cuffs are described in U. S. Patent 4,081,301 entitled "Method and Apparatus for Continuously Attaching Discrete, Stretched Elastic Strands to Predetermined Isolated Portions of Disposable Absorbent Products", which issued to K. B. Buell on March 28, 1979 which patent is incorporated herein by reference. In a preferred embodiment of the diaper 20, as depicted in Figure 1, the flaps 32 are unitary with the absorptive means 31; the topsheet 38 extending beyond the core waist edges 47 16.25 cm (6.5 inches) while the backsheet 42 terminates 2.5 cm (1.0 inch) beyond the core waist edges 47 where it is joined directly to the topsheet 38.

It is also necessary that the diaper 20 have a means for holding the diaper on the wearer. A number of fastening means can be used to hold the diaper on the wearer as are well known in the diaper art. A preferred fastening means, especially for adult size garments, is the suspension system disclosed in U.S. Patent 4,315,508, which issued to M. E. Bolick on February 16, 1982. A preferred fastening means 80, as depicted in Figure 1, has buttonholes 82 adjacent each corner of the diaper 20 and a pair of elastic strips 84 having at each end buttons 86 for use in association with buttonholes 82. Alternatively, tape tab fasteners can be applied to the back upper waist region of the diaper 20 to provide a fastening means to hold the diaper on the wearer. The tape tab fasteners can be any of those well known in the art, such as the fastening tape disclosed in U.S. Patent 3,848,594, which issued to K. B. Buell on November 19, 1974. The suspension systems, or tape tab fasteners, or other diaper fastening means, such as pins, are typically applied near the top edge of a diaper in its "in-use" configuration.

The diaper 20 can also be divided into regions. The diaper 20 has front and back upper waist regions 21 and 22, respectively, each comprising those portions of the diaper 20 which extend outwardly from the core waist edges 47, or in other words, the flaps 32. The upper waist regions comprise those portions of the diaper 20 which, when worn, encircle the upper waist of the wearer. The diaper 20 also has front and back lower waist regions 23 and 24, respectively, each extending inwardly from the core waist edges 47 toward the lateral centerline 34 of the diaper 20 a distance from 1/4 to 1/3 the length of the absorptive means 31. The lower waist regions comprise those portions of the diaper 20 which, when worn, encircle the lower waist of the wearer. The diaper 20 also has a crotch region 26 which is that portion of the diaper 20 between the lower waist regions 23 and 24, and comprises that portion of the diaper 20 which, when worn, is positioned between the legs of the wearer and covers the lower torso of the wearer. The longitudinal centerline of the diaper 20 is designated 35.

In use, the diaper 20 is positioned between the wearer's legs so that the crotch region 26 covers the lower torso, the front waist regions 23 and 21 are positioned adjacent to the front waist area of the wearer and cover the front waist, and the back regions 24 and 22 are positioned adjacent to the back waist area of the wearer and cover the back waist. An elastic strip 84 is then brought across each hip of the wearer so that the buttons 86 can be inserted into the buttonholes 82 thereby forming a fastening means 80 for holding the diaper 20 on the wearer. In this position, the flaps 32 of the diaper 20 are adjacent the wearer's front and back upper waist areas. The diaper 20 has improved breathability in these upper waist areas due to the fact that the flaps 32 have air pervious zones 33 which allow air, vapor, and other gases to pass from the interior of the diaper 20 and the wearer's skin to the exterior of the garment and the surrounding air. The result is that the wearer remains cooler and dryer and hence, more comfortable.

An alternatively preferred embodiment of the diaper 20 is shown in Figure 4. In this embodiment, the backsheet 42 extends 16.25 cm (6.5 inches) beyond the core waist edges 47 of the absorptive means 31, while the topsheet 38 terminates 2.5 cm (1.0 inch) beyond the core waist edges 47 of the absorptive means 31 where it is associated with the backsheet 42. The flaps 32 comprise those parts of the backsheet 42 and the topsheet 38 which extend beyond the core waist edges 47 of the absorptive means 31. The flaps 32 (backsheet 42) each have an air pervious zone 33.

In another alternatively preferred embodiment, as shown in Figure 5, the topsheet 38 and the backsheet 42 of the absorptive means 31 extend beyond the core waist edges 47, each terminating 2.5 cm (1.0 inch) beyond the core waist edges 47 where they are associated together. An overlay sheet 39 is joined to the absorptive means 31 adjacent the garment surface of the backsheet 42. The overlay sheet 39 is continuous between the core waist edges 47 of the absorptive means 31 and it extends beyond each of the core waist edges 47 of the absorptive means 31. As used herein, the word "continuous" is intended to include embodiments wherein the overlay sheet 39 is unbroken and unitary between the core waist edges 47. In an alternatively preferred embodiment, the overlay sheet 39 extends 16.25 cm (6.5 inches) beyond the core waist edges 47 of the absorptive means 31. The flaps 32 comprise the overlay sheet 39, and those parts of the topsheet 38 and the backsheet 42 which extend beyond the core waist edges 47 of the absorptive means 31. The flaps 32 (overlay sheet 39) each have an air pervious zone 33.

In still another alternatively preferred embodiment, as shown in Figure 6, the topsheet 38 and the backsheet 42 of the absorptive means 31 extend beyond the core waist edges 47, each terminating 2.5 cm (1.0 inch) beyond the core waist edges 47 where they are associated together. Separate sheets 37 having distal ends 72 and proximal ends 70 are joined to the absorptive means 31 adjacent the garment surface of the backsheet 42 in such a way that the proximal ends 70 of the separate sheets 37 are located between the core waist edges 47 of the absorptive means 31 and the distal ends 72 of the separate sheets 37 extend 16.25 cm (6.5 inches) beyond the core waist edges 47 of the absorptive means 31. As used herein, the words "located between the core waist edges" are intended to encompass embodiments wherein the proximal ends 70 of the separate sheets 37 are between or adjacent to the core waist edges 47 of the absorptive means 31. The flaps 32 comprise the separate sheets 37 and those parts of the topsheet 38 and the backsheet 42 which extend beyond the core waist edges 47 of the absorptive means 31. The flaps 32 (separate sheets 37) each have an air pervious zone 33.

Overlay sheet 39 and separate sheets 37 are preferably attached to the backsheet 42 of the absorptive means 31 by attachment means (not shown) such as those well known in the art. For example, overlay sheet 39 and separate sheets 37 may be secured to the backsheet 42 by a uniform continuous layer of adhesive, a patterned layer of adhesive, or any array of separate lines or spots of adhesive. Adhesives which have been found to be satisfactory are manufactured by Eastman Chemical Products Company of Kingsport, Tennessee and marketed under the tradename Eastobond A-3.

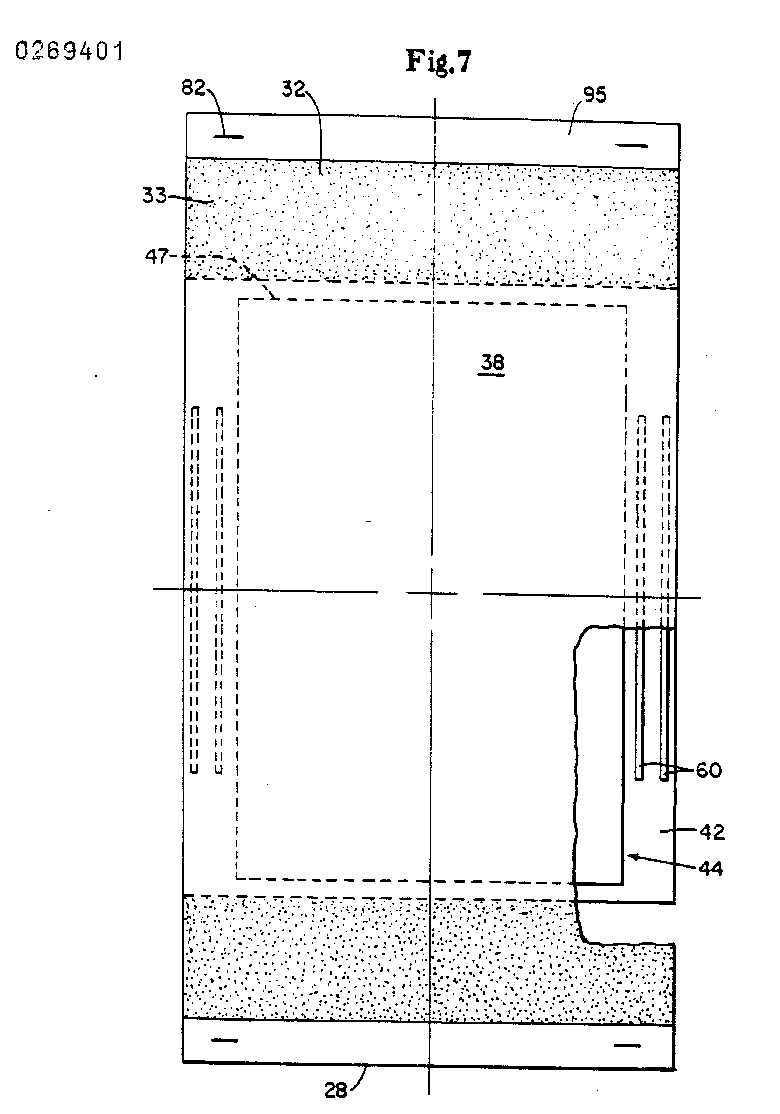

Yet another alternatively preferred embodiment of the diaper 20 is depicted in Figure 7. In this embodiment, the air pervious zones 33 of the flaps 32 do not extend to the furthest extending ends of the flaps 32, or in other words, the end edges 41 of the periphery 28, but instead the air pervious zones 33 are centrally located in the flaps 32 and extend across the width of the flaps 32. Non-pervious poly-tape reinforcing members 95 are added to the furthest extending ends of the flaps 32 to increase the strength and integrity of the diaper 20 during use.

While particular embodiments of the present invention have been illustrated and described, it would be obvious to those skilled in the art that various other changes and modifications can be made without departing from the spirit and scope of the invention. For example, the backsheet 42 of the absorptive means 31 need only be greater than 85% of the length of the absorbent core 44. Alternatively, the flaps 32 preferably extend at least 2.5 cm (1.0 inch), or greater than 2.5 cm (1.0 inch), or at least 7.5 cm (3.0 inches), or at least 12.5 cm (5.0 inches), or at least 17.5 cm (7.0 inches), or at least 22.5 cm (9.0 inches), or at least 27.5 cm (11.0 inches) beyond the core waist edges 47 of the absorptive means 31. Alternatively, overlay sheet 39 and separate sheets 37 may not be joined to the garment surface of the backsheet 42 of the diaper 20, but might be disposed anywhere throughout the layers of the diaper 20. Also, alternatively, there may be only one flap 32, or one flap 32 might be of a different size or extend further than the other flap 32.

## Claims

1. An integral disposable absorbent garment, comprising an absorptive means for absorbing and containing body exudates, said absorptive means having two core waist edges; <u>characterised in that</u> the garment comprises at least one flap joined to and extending from said absorptive means at said core waist edge, said flap having an air pervious zone, the air porosity of which is at least 400 l/sec/m$^2$.

2. An integral disposable absorbent garment according to claim 1, wherein said flap extends beyond one of said core waist edges by at least 2.5 cm.

3. An integral disposable absorbent garment according to either one of claims 1 and 2, wherein said air pervious zone of said flap has an area of from 6.5 to 455.0 square centimeters.

4. An integral disposable absorbent garment according to claim 3, wherein said air pervious zone of said flap has an area of at least 58.5, preferably at least 195.0 square centimeters.

5. An integral disposable absorbent garment according to any one of claims 1-4, wherein said air pervious zone of said flap has an air porosity of at least 800 l/sec/m$^2$, preferably at leat 2000 l/sec/m$^2$.

6. An integral disposable absorbent garment according to any one of claims 1-5, wherein said absorptive means comprises:
   a. a liquid permeable topsheet;
   b. a liquid impermeable backsheet associated with said topsheet; and
   c. an absorbent core positioned between said topsheet and said backsheet.

7. An integral disposable absorbent garment according to claim 6, wherein said flap comprises said topsheet.

8. The integral disposable absorbent garment according to either one of claims 6 and 7, wherein the length of said backsheet is greater than 85% of the length of said absorbent core.

9. An integral disposable absorbent garment according to any one of claims 1-5, wherein said flap comprises an overlay sheet, said overlay sheet being joined to said absorptive means such that said overlay sheet extends longitudinally beyond each of said core waist edges of said absorptive means.

10. An integral disposable absorbent garment according to any one of claims 1-5, wherein said flap comprises a separate sheet, said separate sheet having a distal end and a proximal end, and said separate sheet being joined to said absorptive means such that said proximal end of said separate sheet is located between said core waist edges of said absorptive means and said distal end of said separate sheet is located beyond one of said core waist edges of said absorptive means.

**Fig.1**

0269401

0269401

**Fig.2**

**Fig.3**

**Fig.4**

**Fig.5**

**Fig.6**

0269401

**Fig.7**